# EUROPEAN PATENT APPLICATION

(11) **EP 4 310 718 A1**
(43) Date of publication of application: **24.01.2024**
(21) Application number: 22770967.2
(22) Date of filing: 14.02.2022
(51) Int. Cl.: G06F 30/27, G06F 30/12

(54) **UNIQUE MATERIAL DETECTION DEVICE, CONTROL METHOD, AND NON-TRANSITORY COMPUTER-READABLE MEDIUM**

(30) Priority: 18.03.2021 JP 2021044487
(71) Applicant: NEC Corporation, 108-8001 Tokyo (JP); Tohoku University, Sendai-shi, Miyagi 980-8577 (JP)
(72) Inventor: KUWAMORI Naoki, Tokyo 108-8001 (JP); MUSA Akihiro, Tokyo 108-8001 (JP); TAKIGAWA Yohei, Tokyo 108-8001 (JP); KAZAMA Yuka, Tokyo 108-8001 (JP); SATOU Yoshihiko, Tokyo 136-8627 (JP); KOBAYASHI Hiroaki, Sendai-shi, Miyagi 980-8577 (JP); KIKUGAWA Gota, Sendai-shi, Miyagi 980-8577 (JP); OKABE Tomonaga, Sendai-shi, Miyagi 980-8577 (JP); KOMATSU Kazuhiko, Sendai-shi, Miyagi 980-8577 (JP)
(74) Representative: Betten & Resch
(86) International application number: PCT/JP2022/005625
(87) International publication number: WO 2022/196208

(57) **Abstract**

A singular material detection apparatus (2000) acquires, for each of a plurality of materials (60), material specification information (10) representing material specifications of the material (60) and physical property information (20) indicating physical properties of a product (70) that can be generated by using the material (60). The singular material detection apparatus (2000) generates a self-organizing map (30) by using the physical property information (20). The singular material detection apparatus (2000) assigns each of pieces of material specification information (10) to one of nodes based on the physical property information (20) corresponding to that piece of the material specification information (10). Then, the singular material detection apparatus (2000) detects, from among the nodes to which the pieces of material specification information (10) are assigned, a singular node located at a singular position in a map space.

## Description

### Technical Field

The present disclosure relates to a technique for providing information related to product development.

### Background Art

In product development, it is useful to understand a relationship between a material and a product. Therefore, a system for helping a user or the like understand a relationship between a material and a product has been developed. For example, Patent Literature 1 discloses a system for helping a user or the like understand a causal relationship between design values of a tire and physical property values thereof by using a self-organizing map.

### Citation List

### Patent Literature

Patent Literature 1: Japanese Unexamined Patent Application Publication No. 2016-148988

### Summary of Invention

### Technical Problem

In Patent Literature 1, the self-organizing map is used to determine which one of a plurality of design variables of the tire is important factor. Therefore, it is not assumed that the self-organizing map is used for any purpose other than the above-described purpose. The present disclosure has been made in view of the above-described problem, and an objective thereof is to provide a new technique for providing information useful for product development.

### Solution to Problem

A singular material detection apparatus according to the present disclosure includes: acquisition means for acquiring, for each of a plurality of patterns of a material that can be used in a target process, material specification information representing a material specification of the material and physical property information indicating a physical property quantity for each of a plurality of physical properties of a product that can be generated in the target process by using the material; self-organizing map generation means for generating, by using the physical property information, a self-organizing map on which each node is assigned a position in a map space and a physical property vector indicating a value related to a physical property quantity for each of a plurality of types of the physical properties of the product; and singular node detection means for assigning each material specification information to one of the nodes based on the physical property information corresponding to that material specification information, and detecting a singular node located at a singular position in the map space out of the nodes that are assigned the material specification information.

A control method according to the present disclosure is performed by a computer. The control method includes: an acquisition step of acquiring, for each of a plurality of patterns of a material that can be used in a target process, material specification information representing a material specification of the material and physical property information indicating a physical property quantity for each of a plurality of physical properties of a product that can be generated in the target process by using the material; a self-organizing map generation step of generating, by using the physical property information, a self-organizing map on which each node is assigned a position in a map space and a physical property vector indicating a value related to a physical property quantity for each of a plurality of types of the physical properties of the product; and a singular node detection step of assigning each material specification information to one of the nodes based on the physical property information corresponding to that material specification information, and detecting a singular node located at a singular position in the map space out of the nodes that are assigned the material specification information.

A non-transitory computer readable medium according to the present disclosure stores a program for causing a computer to perform a control method according to the present disclosure.

### Advantageous Effects of Invention

According to the present disclosure, a new technology for providing information useful for product development is provided.

### Brief Description of Drawings

Fig. 1 shows an example of an overview of operations performed by a singular material detection apparatus according to a first example embodiment;
Fig. 2 is a block diagram showing an example of a functional configuration of the singular material detection apparatus according to the first example embodiment;
Fig. 3 is a block diagram showing an example of a hardware configuration of a computer that implements a singular material detection apparatus;
Fig. 4 is a flowchart showing an example of a flow of processes performed by the singular material detection apparatus according to the first example embodiment;
Fig. 5 shows an example of material specification information in the form of a table;
Fig. 6 shows an example of physical property information in the form of a table;
Fig. 7 shows an example of a structure of a self-organizing map on which pieces of material specification information have already been assigned in the form of a table;
Fig. 8 shows an example of a visualized map space;
Fig. 9 shows an example of a case where a singular node is detected from each of a plurality of clusters;
Fig. 10 shows an example of a map image in which a singular node is highlighted; and
Fig. 11 shows an example of a map image in which information about material specifications and physical properties corresponding to a singular node are included.

### Example Embodiment

An example embodiment according to the present disclosure will be described hereinafter in detail with reference to the drawings. Further, components corresponding to or the same as each other are assigned the same or corresponding numerical numbers (or symbols) throughout the drawings, and redundant descriptions thereof are omitted as appropriate. Further, unless otherwise described, predefined information suchas predetermined values and thresholds are stored in advance in a storage device or the like accessible from an apparatus that uses these values.

### [First Example Embodiment]

### <Overvi ew>

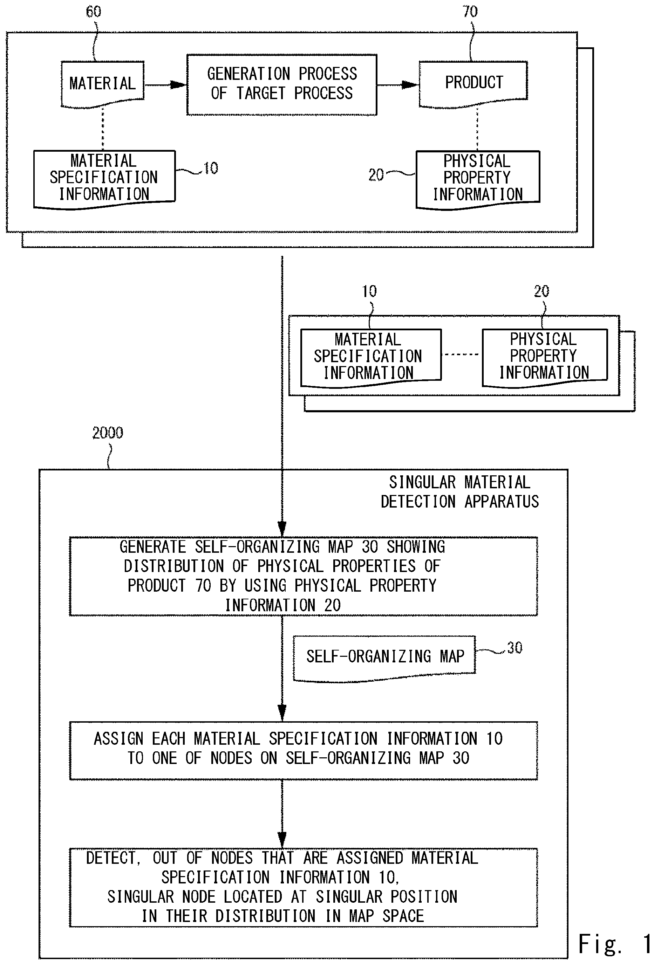

Fig. 1 shows an example of an overview of operations performed by a singular material detection apparatus 2000 according to a first example embodiment. Note that Fig. 1 is a diagram merely for facilitating the understanding of the overview of the singular material detection apparatus 2000, and the operations performed by the singular material detection apparatus 2000 are not limited to those shown in Fig. 1.

The singular material detection apparatus 2000 is used to detect, out of various types of materials 60 that can be used in a specific process (hereinafter also referred to as a target process) in product development, a material 60 with which a product 70 having a singular physical property can be generated. The product 70 is a product that is predicted to be generated or actually generated by processing a material 60 in a generation process of the target process. The material 60 is a material used to generate the product 70. Various patterns of materials 60 can be used in the target process. The physical properties of the product 70 can vary depending on the used material 60.

A pattern of the material 60 is specified by its material specification. In other words, materials 60 having material specifications different from each other are handled as the materials 60 of different pattens from each other. On the other hand, materials 60 having the same material specification as each other are handled as the material 60 of the same pattern as each other.

A material specification is represented by, for example, a type of the material, types of substances constituting the material, a blending ratio of each substance, or a type of processing performed to generate the material. Examples of types of materials include carbon fiber reinforced plastics and stainless steel. For example, assume that the material 60 is a carbon fiber reinforced plastic. In this case, the material specification of the material 60 includes the type of each of one or more carbon fibers that constitute the material 60 (such as polyacrylonitrile fibers and cellulose carbonized fibers), the type of each of one or more of resins that constitute the material 60 (such as epoxy and polyether tephthalate), and the blending ratio of those materials. Further, the material specification may also include a type of fiber directional polymerization method, a type of crimping method, or a resin composition.

The singular material detection apparatus 2000 operates as described hereinafter. That is, the singular material detection apparatus 2000 acquires, for each of a plurality of various patterns of the material (in other words, for materials 60 specified by respective material specifications of various patterns), material specification information 10 representing the material specification of the material 60 and physical property information 20 indicating physical properties of a product 70 that can be generated in the target process by using the material 60. The physical property information 20 indicates a physical property quantity for each of a plurality of types of physical properties of the product 70. Examples of types of physical properties include incombustibility, heat resistance, elastic modulus, or tenacity.

The singular material detection apparatus 2000 generates a self-organizing map 30 showing a distribution of physical properties of the product 70 by using the physical property information 20. The self-organizing map 30 has a plurality of nodes arranged in an m-dimensional space. Hereafter, this m-dimensional space is referred to as a map space. As will be described hereinafter, when the singular material detection apparatus 2000 generates an image in which the arrangement of nodes in the map space is visualized, m is set to two or three. In this case, for example, nodes can be represented by cells of a checkered pattern or grid points of a grid pattern.

Each of the nodes on the self-organizing map 30 is assigned multi-dimensional data (hereinafter also referred to as a physical property vector) that represents the magnitude of the physical property quantity for each of a plurality of types of the physical properties. For example, assume that four types of physical properties including incombustibility, heat resistance, elastic modulus, and tenacity, are used. In this case, the physical property vector is a four-dimensional data that represents the magnitude of the physical property quantity for each of these four types of physical properties. In the following description, the number of dimensions of the physical property vector is denoted by n. Note that n is larger than m (n>m). That is, on the self-organizing map 30, the space of the physical property vector is a high-dimensional space while the map space is a low-dimensional space.

The physical property information 20 indicates physical property quantities of at least n types of physical properties. The singular material detection apparatus 2000 performs training for the self-organizing map 30 by using physical property quantities of the n types of physical properties indicated by the physical property information 20 and determines a physical property vector to be assigned to each of the nodes, thereby generating a self-organizing map 30.

Further, the singular material detection apparatus 2000 assigns each of a plurality of pieces of material specification information 10 to one of the nodes on the self-organizing map 30. Specifically, a singular node detection unit 2060 determines a node having a physical property vector that is most similar to a physical property vector obtained from the physical property information 20 corresponding to the material specification information 10. Then, the singular node detection unit 2060 assigns the material specification information 10 to the determined node.

The singular material detection apparatus 2000 detects, out of the nodes which are associated with the material specification information 10, a node that is located at a singular position in the distribution of these nodes in the map space as a singular node. This means that the position of the singular node is a statistically singular position (i.e., is an outlier) in the distribution of the nodes in the map space that are associated with the material specification information 10.

### <Example of Advantageous Effect>

When a product is developed through trial and error, there are rare cases where a product having a singular physical property is obtained. Such a singular product is considered important because it can lead to the discovery of a new principle, a new additive, or a new property through its research.

According to the singular material detection apparatus 2000, it is possible to detect a material 60 with which a singular product can be generated. Specifically, the singular material detection apparatus 2000 generates a self-organizing map 30 using physical property information 20. As a result, a self-organizing map 30 on which a distribution of physical properties is shown by the arrangement of nodes in a map space. Further, the singular material detection apparatus 2000 assigns each of pieces of the material specification information 10 to a node on the self-organizing map 30, and detects a singular node out of the nodes that are assigned the material specification information 10. The singular node is, among the nodes that are assigned the material specification information 10, a node located largely distant from other nodes.

Note that the fact that a distance between two nodes is large in a map space means that the degree of similarity between physical properties of these nodes is low. Thus, it can be considered that a material 60 represented by the material specification information 10 assigned to a singular node is a material 60 with which a product 70 having a singular physical property can be generated. Therefore, a user of the singular material detection apparatus 2000 can find a material 60 with which a singular product can be generated, by using the singular material detection apparatus 2000.

The singular material detection apparatus 2000 according to this example embodiment will be described hereinafter in a more detailed manner.

### <Example of Functional Configuration>

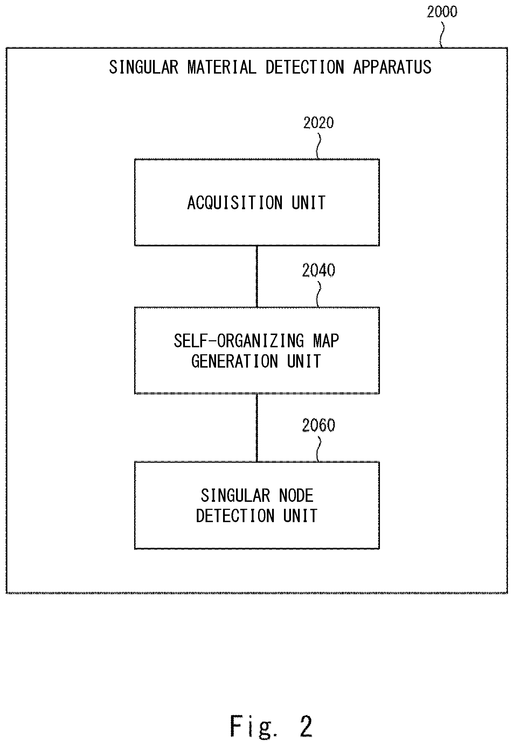

Fig. 2 is a block diagram showing an example of a functional configuration of the singular material detection apparatus 2000 according to the first example embodiment. The singular material detection apparatus 2000 includes an acquisition unit 2020, a self-organizing map generation unit 2040, and a singular node detection unit 2060. The acquisition unit 2020 acquires material specification information 10 and physical property information 20 for each of a plurality of various patterns of the material 60. The self-organizing map generation unit 2040 generates a self-organizing map 30 by using the physical property information 20. The singular node detection unit assigns each of pieces of material specification information 10 to a respective one of the nodes on the self-organizing map 30. Further, the singular node detection unit 2060 detects, out of the nodes that are assigned the material specification information 10, a node that is located at a singular position in the distribution of these nodes in the map space as a singular node.

### <Example of Hardware Configuration>

Each of functional components of the singular material detection apparatus 2000 can be implemented by hardware that implements the functional component (e.g., a hardwired electronic circuit or the like) or by a combination of hardware and software (e.g., a combination of an electronic circuit and a program for controlling it or the like). A case where each of the functional components of the singular material detection apparatus 2000 is implemented by a combination of hardware and software will be further described hereinafter.

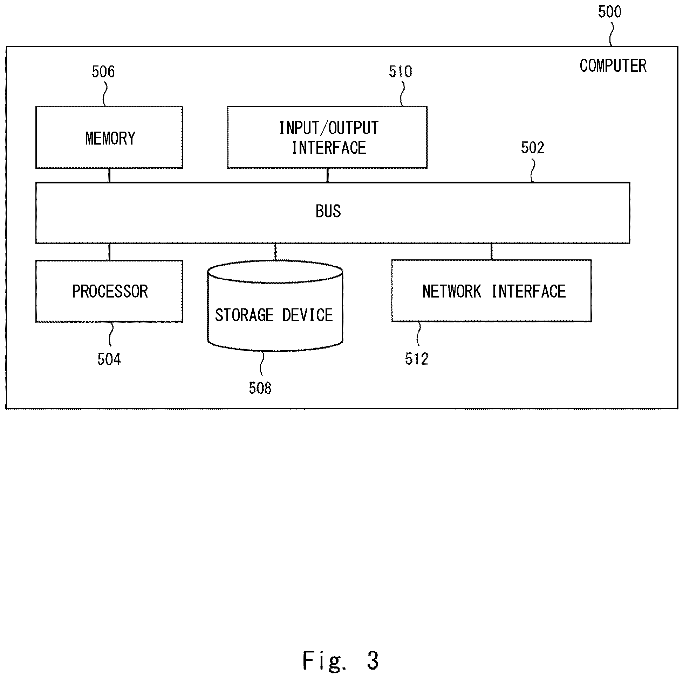

Fig. 3 is a block diagram showing an example of a hardware configuration of a computer 500 that implements the singular material detection apparatus 2000. The computer 500 is an arbitrary computer. For example, the computer 500 is a stationary computer such as a server machine or a PC (Personal Computer). Alternatively, for example, the computer 500 is a portable computer such as a smartphone or a tablet-type terminal. The computer 500 may be a special-purpose computer designed to realize the singular material detection apparatus 2000, or may be a general-purpose computer.

For example, each of functions of the singular material detection apparatus 2000 is implemented by the computer 500 by installing a predetermined application in the computer 500. The aforementioned application is composed of a program for implementing each of the function components of the singular material detection apparatus 2000. Note that how to acquire the aforementioned program is arbitrarily determined. For example, the program can be acquired from a storage medium (such as a DVD or a USB memory) in which the program is stored. Alternatively, the program can be acquired, for example, by downloading the program from a server apparatus that manages a storage device in which the program is stored.

The computer 500 includes a bus 502, a processor 504, a memory 506, a storage device 508, an input/output interface 510, and a network interface 512. The bus 502 is a data transmission path through which the processor 504, the memory 506, the storage device 508, the input/output interface 510, and the network interface 512 transmit and receive data to and from each other. However, the method for connecting the processor 504 and the like to each other is not limited to connections through buses.

The processor 504 is any of various types of processors such as a CPU (Central Processing Unit), a GPU (Graphics Processing Unit), or an FPGA (Field-Programmable Gate Array). The memory 506 is a primary storage device implemented by using a RAM (Random Access Memory) or the like. The storage device 508 is a secondary storage device implemented by using a hard disk drive, an SSD (Solid State Drive), a memory card, or a ROM (Read Only Memory).

The input/output interface 510 is an interface for connecting the computer 500 with an input/output device(s). For example, an input device such as a keyboard and an output device such as a display device are connected to the input/output interface 510.

The network interface 512 is an interface for connecting the computer 500 to a network. The network may be a LAN (Local Area Network) or a WAN (Wide Area Network).

In the storage device 508, programs for implementing respective functional components of the singular material detection apparatus 2000 (programs for implementing the above-described applications) are stored. The processor 504 implements each of functional components of the singular material detection apparatus 2000 by loading the aforementioned program onto the memory 506 and executing the loaded program.

The singular material detection apparatus 2000 may be implemented by one computer 500 or by a plurality of computers 500. In the latter case, the configurations of the computers 500 do not need to be identical to each other, but can be different from each other.

### <Flow of Processes>

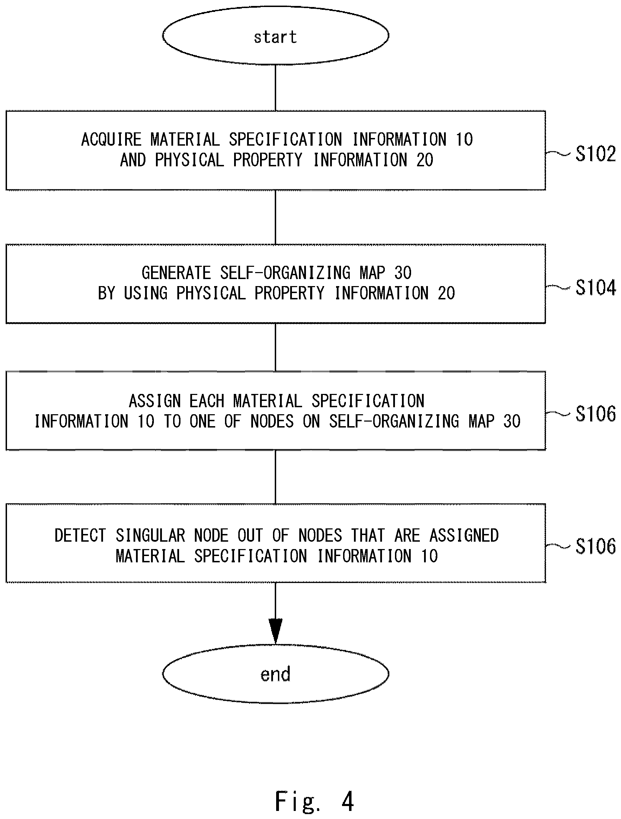

Fig. 4 is a flowchart showing an example of a flow of processes performed by the singular material detection apparatus 2000 according to the first example embodiment. The acquisition unit 2020 acquires material specification information 10 and physical property information 20 for each of a plurality of various pattens of the materials 60 (S102). The self-organizing map generation unit 2040 generates a self-organizing map 30 by using the physical property information 20 (S104). The singular node detection unit 2060 assigns each of pieces of the material specification information 10 to one of the nodes on the self-organizing map 30 (S106). The singular node detection unit 2060 detects, out of the nodes that are assigned the material specification information 10, a node that is located at a singular position in the distribution of these nodes in the map space as a singular node (S108).

### <Acquisition of Material Specification Information 10 and Physical Property Information 20: S102>

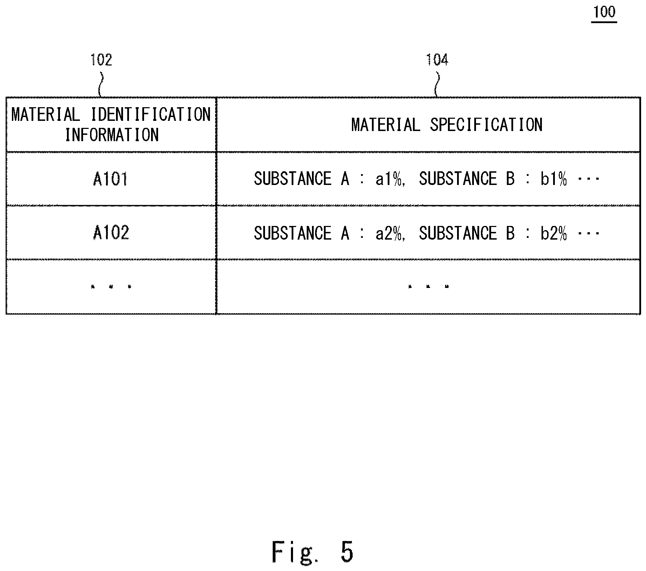

For each of a plurality of various patterns of the material 60 that can be used in the target process, the acquisition unit 2020 acquires material specification information 10 representing the material specification of that material 60 and physical property information 20 for a product 70 that can be generated by using the material 60 (S102). Fig. 5 shows an example of the material specification information 10 in the form of a table. Table 100 in Fig. 5 has a column named Material Identification Information 102 and a column named Material Specification 104. The material identification information 102 indicates identification information assigned to a material 60. The material specification 104 indicates a specification of the material 60.

In Fig. 5, the material specification information 10 is represented by one record in Table 100. That is, the material specification information 10 associates the identification information of a material 60 and the material specification of the material 60 having that identification information.

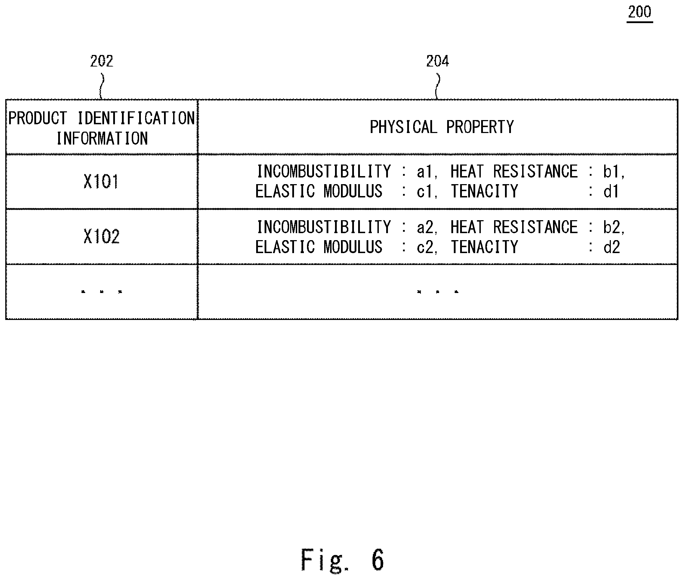

Fig. 6 shows an example of the physical property information 20 in the form of a table. Table 200 in Fig. 6 has a column named Product Identification Information 202 and a column named Physical Property 204. The product identification information 202 indicates identification information of a product 70. The physical property 204 indicates physical properties of the product 70. In Table 200, a physical property of the product 70 is represented by indicating an association "Label indicating Type of Physical Property: Physical Property Quantity of the Physical Property".

In Fig. 6, the physical property information 20 is represented by one record in Table 200. That is, the physical property information 20 associates the identification information of a product 70 with the physical properties of the product 70 having that identification information.

The acquisition unit 2020 acquires a plurality of pairs of the material specification information 10 and the physical property information 20. There are various methods by which the acquisition unit 2020 acquires the pairs of the material specification information 10 and the physical property information 20. For example, pairs of the material specification information 10 and the physical property information 20 are stored in advance in an arbitrary storage device accessible from the singular material detection apparatus 2000. The acquisition unit 2020 acquires a pair of the material specification information 10 and the physical property information 20 by accessing this storage device. Alternative, for example, the acquisition unit 2020 may acquire a pair of the material specification information 10 and the physical property information 20 by receiving an input from a user for entering the pair of the material specification information 10 and the physical property information 20. Alternatively, for example, the acquisition unit 2020 may acquire a pair of the material specification information 10 and the physical property information 20 by receiving the pair of the material specification information 10 and the physical property information 20 transmitted from other apparatuses.

Note that there are various methods for generating a pair of the material specification information 10 and the physical property information 20. For example, a pair of the material specification information 10 and the physical property information 20 is generated by performing a simulation of the generation of a product 70. Specifically, by performing a simulation given an input of a specific material specification, physical property information 20 that indicates a predicted physical property quantity of each physical property of a product 70 is generated. Then, a pair of the generated physical property information 20 and the material specification information 10 indicating the material specification given as the input is obtained. Note that an existing technique can be used for the above-described technique in which a material specification is acquired as an input and a simulation for outputting predicted data of physical properties of a product that is generated in a specific process using a material specified by the acquired material specifications is performed.

Alternatively, for example, a pair of the material specification information 10 and the physical property information 20 can be generated by actually producing a product 70. Specifically, a product 70 is experimentally generated by using a material 60 represented by a specific material specification in the target process. Further, physical property information 20 is generated by measuring a physical property quantity of each of physical properties of the generated product 70. As a result, a pair of the generated physical property information 20 and the material specification information 10 representing the utilized material 60 is obtained.

Note that a plurality of pieces of physical property information 20 acquired by the acquisition unit 2020 may include those that express data in different ways from each other. For example, it is conceivable that different labels are used for physical properties that are essentially the same as each other. Further, it is conceivable that physical property quantities of the same physical property are expressed in units different from each other. In such a case, it is preferred that the acquisition unit 2020 unifies the ways of expressing data, for example, by unifying labels or performing inter-unit conversion. It is conceivable that such a situation in which the ways of expressing data of pieces of physical property information 20 are different from each other could occur, for example, when pieces of physical property information 20 generated by using a simulation and pieces of physical property information 20 generated by actually generating a product 70 are both acquired. Note that it is preferred that the unification of ways of expressing data is also carried out for the material specification information 10 in a similar manner.

### <Generation of Self-Organizing Map 30: S104>

The self-organizing map generation unit 2040 generates a self-organizing map 30 by using the physical property information 20 (S104). The self-organizing map 30 has a plurality of nodes arranged in an m-dimensional map space. The number of dimensions of the map space may be determined in advance or designated by a user.

Each of the nodes on the self-organizing map 30 is assigned an n-dimensional physical property vector. The assignment of a physical property vector to each node is carried out through the training of the self-organizing map 30. The training of the self-organizing map 30 can be carried out by inputting n-dimensional training data to be used for the training into the self-organizing map 30. Note that an existing method can be used as an actual method for training the self-organizing map by using training data.

For example, the self-organizing map generation unit 2040 initializes the self-organizing map 30 by an arbitrary method. As the initialization method, for example, a method to initialize a physical property vector of each node to a random value can be adopted. The self-organizing map generation unit 2040 extracts a physical property quantity for each of the n-types of the physical properties from each of the acquired pieces of the physical property information 20 to generate n-dimensional physical property vectors. The self-organizing map generation unit 2040 performs the training of the self-organizing map 30 using each of the physical property vectors as training data, thereby generating the self-organizing map 30. As a result, a physical property vector of each node on the self-organizing map 30 becomes n-dimensional data indicating a value for each of the physical property quantities of n types of the physical properties.

The physical property vector obtained from the physical property information 20 may indicate each of physical property quantities of the n types of the physical properties represented by the physical property information 20 as it is, or may indicate a value that is obtained by converting each of physical property quantities with a predetermined method (e.g., normalization, standardization, or the like).

Note that the number of physical properties represented by the physical property information 20 may be greater than n. In this case, some of data represented by the physical property information 20 are used to generate the self-organizing map 30. Note that which types of the physical properties represented by the physical property information 20 are used to generate the self-organizing map 30 may be determined in advance or designated by a user.

### <Assignment of Material Specification Information 10: S106>

The singular node detection unit 2060 assigns each of pieces of the material specification information 10 acquired by the acquisition unit 2020 to one of the nodes on the self-organizing map 30 (S106). Specifically, the singular node detection unit 2060 determines a node having a physical property vector that is most similar to the physical property vector obtained from the physical property information 20 corresponding to the material specification information 10. Then, the singular node detection unit 2060 assigns the material specification information 10 to the determined node.

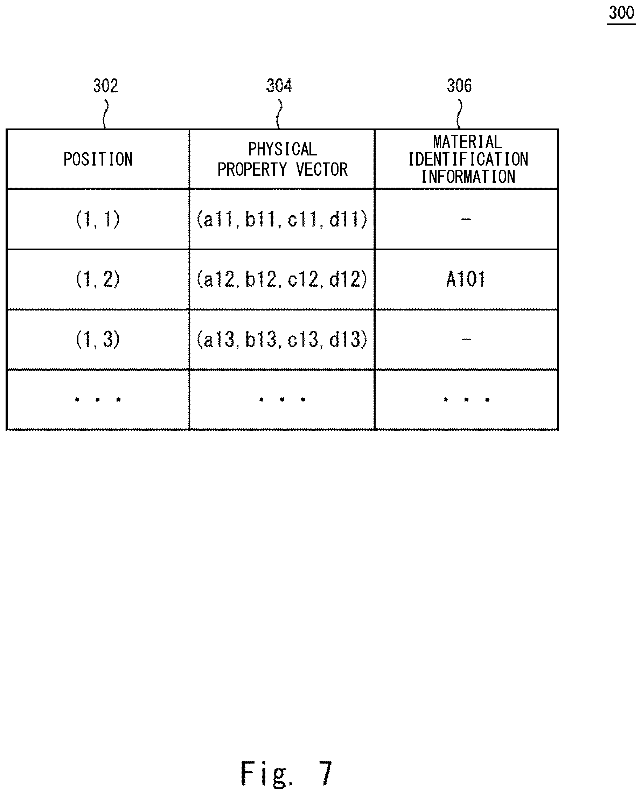

Fig. 7 shows an example of a structure of the self-organizing map 30 on which pieces of the material specification information 10 have already been assigned in the form of a table. Table 300 in Fig. 7 has three columns: Position 302, Physical Property Vector 304, and Material Identification Information 306. Table 300 has one record for one node.

The position 302 indicates coordinates of a node in the m-dimensional space. In the example shown in Fig. 7, m is two (m=2), and x-coordinate and y- coordinate are assigned to a node. The physical property vector 304 shows a n-dimensional physical property vector assigned to the node. In the example shown in Fig. 7, n is four (n=4). For each of nodes to which pieces of material specification information 10 are assigned, the material identification information 306 shows identification information of a material 60 indicated by the piece of material specification information 10 assigned to the node. In a record of a node to which no material specification information 10 is assigned, the material identification information 306 shows "-".

### <Detection of Singular Node: S108>

The singular node detection unit 2060 detects a singular node out of the nodes that are assigned the material specification information 10 (S108). The singular node is, among the nodes that are assigned the material specification information 10, a node that is located at a singular position in the distribution of these nodes in the map space.

For example, the singular node detection unit 2060 determines a reference position and a singularity threshold based on the distribution of the positions of the nodes in the map space that are assigned the material specification information 10, and detects a singular node by using the determined reference position and singularity threshold. The reference position and the singularity threshold are determined so that a node whose distance from the reference position is larger than the singularity threshold, among the nodes that are assigned the material specification information 10, becomes a position that is singular from the statistical point of view in the above-mentioned distribution. Then, the node whose distance from the reference position is larger than the singularity threshold, among the nodes that are assigned the material specification information 10, is detected as a singular node.

For example, the singular node detection unit 2060 computes, based on the position of each node in the map space that is assigned the material specification information 10, the center of mass or the geometric center of these nodes, and uses the computed position as the reference position. Further, the singular node detection unit 2060 computes, based on the position of each node in the map space that is assigned the material specification information 10, a statistical value (such as a standard deviation) indicating the magnitude of the distribution of these nodes, and computes their statistical value or a value that is obtained by multiplying the statistical value by a positive real number as the singularity threshold. Note that existing techniques can be used for a technique for computing, based on a plurality of positions in a multi-dimensional space, the center of mass or the geometric center of these positions, and for a technique for computing a statistical value indicating the magnitude of the distribution of these positions.

For each node that is assigned the material specification information 10, the singular node detection unit 2060 computes a distance between the node and the reference position and determines whether or not the computed distance is larger than the singularity threshold. Then, the singular node detection unit 2060 detects a node whose distance from the reference position is determined to be larger than the singularity threshold as a singular node.

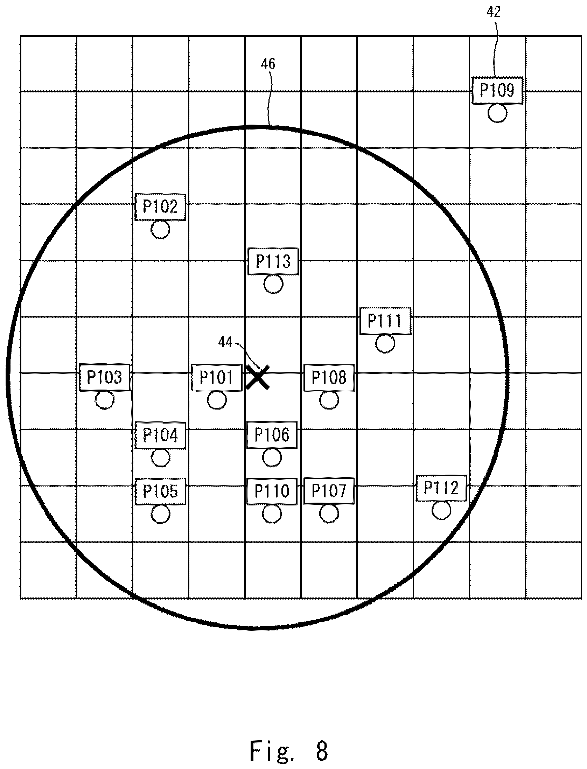

Fig. 8 shows an example of a visualized map space. In the example shown by Fig. 8, the number of dimensions of the map space is two. Each node is represented by a cell of a checkered pattern. An indication 42 shows information by which the material specification information 10 associated with a node can be specified. Specifically, in the example shown by Fig. 8, the indication 42 shows material identification information. A reference position 44 represents the reference position. An area 46 represents a range in which the distance from the reference position 44 is equal to or shorter than the singularity threshold.

In Fig. 8, the node corresponding to the indication 42 indicating P109 is located outside the area 46. That is, the position of this node is a position whose distance from the reference position 44 is larger than the singularity threshold. Therefore, this node is detected as a singular node.

### <Clustering of Node>

In the example shown by Fig. 8, a singular node is detected based on the distribution of all the nodes that are assigned the material specification information 10. In this regard, the singular node detection unit 2060 may divide the nodes that are assigned the material specification information 10 into a plurality of clusters and detect a singular node from each cluster. For example, for each node that is assigned the material specification information 10, the singular node detection unit 2060 assigns multi-dimensional data (hereinafter also referred to as a specification vector) representing a value of each of a plurality of types of parameters indicated by the corresponding material specification information 10 to the node. Then, the singular node detection unit 2060 divides the plurality of nodes that are assigned the material specification information 10 into a plurality of clusters by performing the clustering on them based on the corresponding specification vectors. Note that an arbitrary clustering algorithm such as a k-means method can be used as a method for the clustering.

Note that the specification vector may indicate values of all parameters indicated by the material specification information 10 or may indicate values of some of all the parameters. That is, when the number of dimensions of the specification vector is represented by k, the value of k may be equal to the number of the parameters indicated by the material specification information 10 or smaller than the number of the parameters indicated by the material specification information 10.

For example, assume that the material specification information 10 indicates both parameters that have continuous values (e.g., a blending ratio of a substance) and parameters that do not have continuous values (e.g., a type of processing or the like). In this case, the specifications vector is generated, for example, by using only parameters having continuous values.

Note that which of the parameters indicated by the material specification information 10 are used to generate the specification vector may be determined in advance or designated by a user. Further, the specification vector may indicate the value of a parameter indicated by the material specification information 10 as it is, or may indicate a value that is obtained by converting the value of a respective parameter with a predetermined method (e.g., normalization, standardization, or the like).

The method for the clustering is not limited to methods using specification vectors. For example, the singular node detection unit 2060 may cluster perform the clustering on the nodes based on the type of material indicated by the material specification information 10. For example, the material specification information 10 acquired by the singular material detection apparatus 2000 may include materials 60 of types different from each other, such as including a material 60 that is classified as carbon fiber reinforced plastic and a material 60 that is classified as stainless steel. Therefore, the singular node detection unit 2060 classifies the nodes into clusters according to the type of material 60 indicated by the assigned material specification information 10. In this way, a singular node can be detected for each type of material 60.

Further, after the singular node detection unit 2060 generates a cluster for each type of material 60, it may further apply the clustering to each cluster based on the specification vector.

The singular node detection unit 2060 computes a reference position and a singularity threshold for each of the clusters. Note that it is assumed that the total number of clusters is Nc. Further, an i-th cluster is expressed as a cluster i. Further, a reference position and a singularity threshold computed for the cluster i are expressed as a reference position i and a singularity threshold i, respectively. For example, the singular node detection unit 2060 detects a singular node for each cluster by performing the following process for each of the clusters i (i = 1, 2,..., Nc).

First, the singular node detection unit 2060 computes a reference position i and a singularity threshold i based on the positions of a plurality of nodes included in a cluster i among the nodes that are assigned the material specification information 10. A computation method similar to the method that is used in the case where clustering is not performed can be adopted. That is, for example, the singular node detection unit 2060 computes the position of the center of mass or the geometric center with respect to the positions of a plurality of nodes included in the cluster i among the nodes that are assigned the material specification information 10, and handles the computed position of the center of mass or the like as the reference position i. Further, for example, the singular node detection unit 2060 computes a statistic value representing the magnitude of the distribution of the positions of the plurality of nodes included in the cluster i among the nodes that are assigned the material specification information 10 are assigned, and handles the computed statistical value or a value that is obtained by multiplying the statistical values by a positive real number as the singularity threshold.

Further, for each node included in the cluster i among the nodes that are assigned the material specification information 10, the singular node detection unit 2060 determines whether or not a distance between the position of the node and the reference position i is larger than the singularity threshold i. Then, the singular node detection unit 2060 detects a node whose distance from the reference position i is determined to be larger than the singularity threshold i as a singular node in the cluster i.

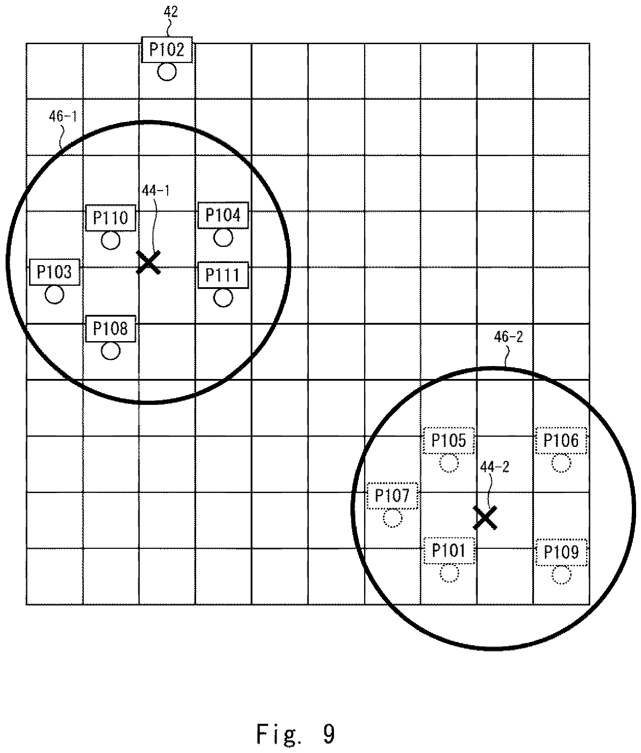

Fig. 9 shows an example of a case where a singular node is detected from each of a plurality of clusters. In the example shown in Fig. 9, nodes that are assigned the material specification information 10 are divided into two clusters. Regarding each node that is classified into a cluster 1, the frame of the indication 42 is represented by a solid line. Meanwhile, regarding each node that is classified into a cluster 2, the frame of the indication 42 is represented by a dotted line.

A reference position 44-1 represents the reference position computed for the cluster 1. Further, an area 46-1 represents a range in which the distance from the reference position 44-1 is equal to or smaller than the singularity threshold computed for the cluster 1. Among the nodes classified in the cluster 1, a node whose material identification information is P102 is located outside the area 46-1. Therefore, this node is detected as a singular node for the cluster 1.

Meanwhile, a reference position 44-2 represents the reference position computed for the cluster 2. Further, an area 46-2 represents a range in which the distance from the reference position 44-2 is equal to or smaller than the singularity threshold computed for the cluster 2. Note that all the nodes classified into the cluster 2 are located within the area 46-2. Therefore, no singular node is detected from the cluster 2.

Advantageous effects that are obtained by performing the above-described clustering will be described hereinafter. When products 70 are generated by using materials 60 whose material specifications are similar to each other, there is a high probability that the generated products 70 will be similar to each other. Therefore, there is a high probability that nodes assigned pieces of material specification information 10 similar to each other are located at positions close to each other in the map space. Therefore, it is considered that if there is a node whose position is singular in the map space among the nodes that are assigned pieces of the material specification information 10 similar to each other, the material specification information 10 assigned to this node represents a singular material 60.

Therefore, the singular material detection apparatus 2000 performs clustering based on the material specifications and detects a singular node for each of the clusters. By doing so, it is possible to detect a material 60 with which a singular product 70 can be generated out of the materials 60 similar to each other.

### <Output of Processing Result>

The singular material detection apparatus 2000 outputs output information representing a result of processing. The output information includes information by which it is possible to know a detected singular node. The information by which it is possible to know a detected singular node includes, for example, an image showing a visualized map space (hereinafter also referred to as a map image) and data of a screen including the image (e.g., a window showing the image). In the following description, a functional component that generates such a map image is referred to as a map image generation unit (not illustrated).

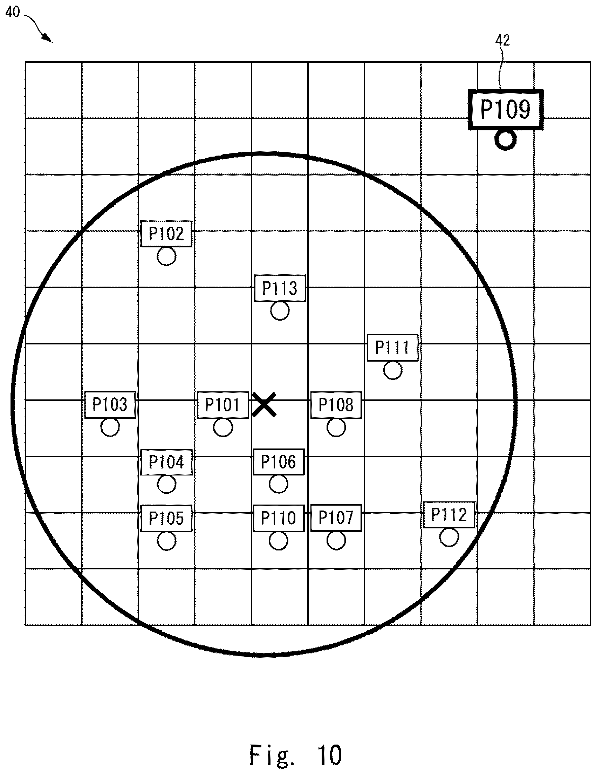

Note that it is preferable that in the map image, a singular node is highlighted so that the singular node can be easily distinguished from the other nodes. Fig. 10 shows an example of a map image in which a singular node is highlighted. Note that the map image 40 shown by Fig. 10 is similar to that shown by Fig. 8, except that the indication 42 corresponding to the singular node is highlighted.

In Fig. 10, the singular node is a node having the material identification information of P109. Therefore, the size of the indication 42 attached to this node is larger that of the other indications 42, and its frame is thicker than those of the other indications 42.

Here, it is assumed that the user of the singular material detection apparatus 2000 is interested in the material specification and the physical properties corresponding to the singular node. Therefore, for example, the map image 40 may include information about the material specification and the physical properties associated with the singular node.

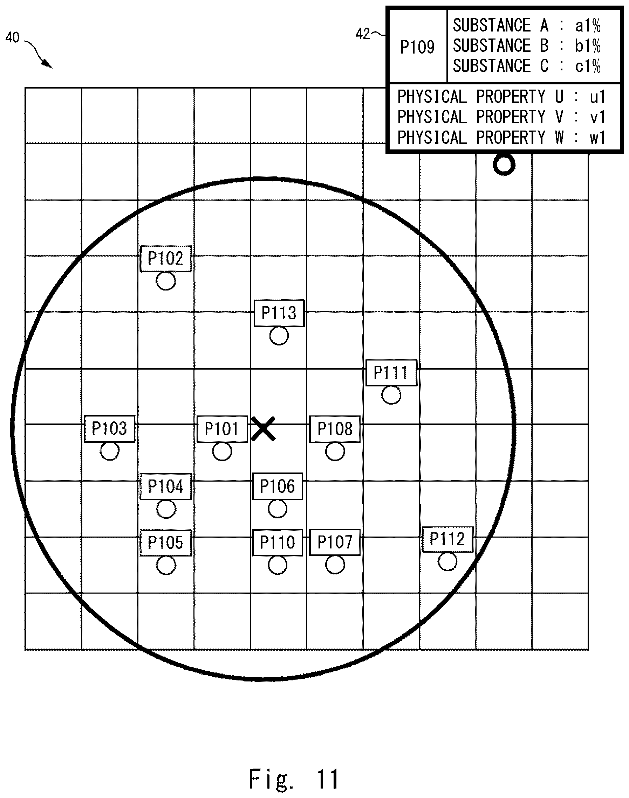

Fig. 11 shows an example of the map image 40 in which information about the material specification and the physical properties corresponding to the singular node are included. What is shown by Fig. 11 is the same as that shown by Fig. 10, except that the contents of the indication 42 are different from those shown by Fig. 10. In Fig. 11, the indication 42 of the singular node shows, as the content of the material specification information 10 corresponding to the singular node, the material identification information of "P109" and the material specification which is blending ratios of substances A to C. Further, the indication 42 of the singular node also shows physical property quantities of physical properties U, V and W, respectively, as the content of the physical properties corresponding to the singular node.

Note that in the case where screen data including the map image 40 is included in the output information, it may be configured so as to receive an input from the user on the screen represented by the screen data and to output information related to the node in response to the input. For example, on a screen that is first output to a display device of a terminal used by a user, the map image 40 shown by Fig. 10 is shown as a default image. That is, the material identification information is used as the content of the indication 42 of each node. After that, when an input operation for designating a specific node (e.g., tapping, clicking, or placing a mouse pointer over the indication 42 of a node) is performed by a user, the indication 42 for that node is changed to an indication 42 showing the material specification and the physical properties as shown in the indication 42 shown by Fig. 11.

Note that the value of each element of a physical property vector assigned to a node may not represent the physical property quantity as it is, but may represent a value obtained by converting it by normalization or the like. In such a case, the singular material detection apparatus 2000 converts the values of elements of the physical property vector of the node designated by the user into actual physical property quantities, and includes the physical property quantities obtained by the conversion into the indication 42.

The output information is not limited to the map image 40 or the screen that includes the map image. For example, the output information may be text information indicating information about the singular node, such as information about its position in the map space, the content of material specification information 10 assigned thereto, and the physical properties of the product 70 represented by the assigned physical property vector. Further, the output information may also indicate the degree of singularity for the singular node based on its position in the map space. For example, the degree of singularity can be represented by a value that is obtained by dividing the distance between the singular node and the reference position by the singularity threshold. This degree of singularity may also be included in the map image 40.

Note that how to output the output information is arbitrarily determined. For example, the singular material detection apparatus 2000 puts the output information in an arbitrary storage device accessible from the singular material detection apparatus 2000. Alternatively, for example, the singular material detection apparatus 2000 displays the output information on an arbitrary display device controllable from the singular material detection apparatus 2000. Alternatively, for example, the singular material detection apparatus 2000 transmits the output information to an arbitrary apparatus that is communicably connected to the singular material detection apparatus 2000.

Although the present invention is described above with reference to example embodiments, the present invention is not limited to the above-described example embodiments. Various modifications that can be understood by those skilled in the art can be made to the configuration and details of the present invention within the scope of the invention.

Note that, in the above-described examples, the program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g., magneto-optical disks), CD-ROM, CD-R, CD-R/W, and semiconductor memories (such as mask ROM, PROM (Programmable ROM), EPROM (Erasable PROM), flash ROM, RAM, etc.). Further, the program may be provided to a computer using any type of transitory computer readable media. Examples of transitory computer readable media include electric signals, optical signals, and electromagnetic waves. Transitory computer readable media can provide the program to a computer via a wired communication line (e.g., electric wires, and optical fibers) or a wireless communication line.

The whole or part of the example embodiments disclosed above can be described as, but not limited to, the following supplementary notes.

### (Supplementary Note 1)

A singular material detection apparatus comprising:
acquisition means for acquiring, for each of a plurality of patterns of a material that can be used in a target process, material specification information representing a material specification of the material and physical property information indicating a physical property quantity for each of a plurality of physical properties of a product that can be generated in the target process by using the material;
self-organizing map generation means for generating, by using the physical property information, a self-organizing map on which each node is assigned a position in a map space and a physical property vector indicating a value related to a physical property quantity for each of a plurality of types of the physical properties of the product; and
singular node detection means for assigning each material specification information to one of the nodes based on the physical property information corresponding to that material specification information, and detecting a singular node located at a singular position in the map space out of the nodes that are assigned the material specification information.

### (Supplementary Note 2)

The singular material detection apparatus according to Supplementary note 1,
wherein for each of a plurality of pieces of the material specification information, the singular node detection means assigns the material specification information to the node to which the physical property vector that is most similar to the physical property vector obtained from the physical property information corresponding to that material specification information is assigned.

### (Supplementary Note 3)

The singular material detection apparatus according to Supplementary note 1 or 2,
wherein the singular node detection means detects, out of the nodes that are assigned the material specification information, the node whose distance from a reference position in the map space is larger than a threshold as a singular node.

### (Supplementary Note 4)

The singular material detection apparatus according to Supplementary note 3,
wherein the singular node detection means performs:
using a center of mass or a geometric center of the plurality of nodes that are assigned the material specification information as the reference position; and
using, as the threshold, a statistic value representing a magnitude of a distribution of positions of the plurality of nodes that are assigned the material specification information are assigned or a value obtained by multiplying the statistic value by a predetermined positive real number.

### (Supplementary Note 5)

The singular material detection apparatus according to any one of Supplementary notes 1 to 4,
wherein the singular node detection means performs:
dividing the plurality of the nodes that are assigned the material specification information into a plurality of clusters based on the material specification information; and
detecting, for each of the plurality of the clusters, the singular node out of the nodes belonging to the cluster based on a distribution of positions of the nodes in the map space included in the cluster.

### (Supplementary Note 6)

The singular material detection apparatus according to any one of Supplementary notes 1 to 5, further comprising map image generation means for generating a map image showing each of the nodes arranged in the map space,
wherein the map image includes an indication indicating the singular node.

### (Supplementary Note 7)

The singular material detection apparatus according to Supplementary note 6,
wherein the indication indicating the singular node indicates one or both of the material specification represented by the material specification information assigned to the singular node and the physical properties represented by the physical property vector assigned to the singular node.

### (Supplementary Note 8)

A control method performed by a computer, comprising:
an acquisition step of acquiring, for each of a plurality of patterns of a material that can be used in a target process, material specification information representing a material specification of the material and physical property information indicating a physical property quantity for each of a plurality of physical properties of a product that can be generated in the target process by using the material;
a self-organizing map generation step of generating, by using the physical property information, a self-organizing map on which each node is assigned a position in a map space and a physical property vector indicating a value related to a physical property quantity for each of a plurality of types of the physical properties of the product; and
a singular node detection step of assigning each material specification information to one of the nodes based on the physical property information corresponding to that material specification information, and detecting a singular node located at a singular position in the map space out of the nodes that are assigned the material specification information.

### (Supplementary Note 9)

The control method according to Supplementary note 8,
wherein in the singular node detection step, for each of a plurality of pieces of the material specification information, assigning the material specification information to the node to which the physical property vector that is most similar to the physical property vector obtained from the physical property information corresponding to that material specification information is assigned.

### (Supplementary Note 10)

The control method according to Supplementary note 8 or 9,
wherein in the singular node detection step, detecting, out of the nodes that are assigned the material specification information, the node whose distance from a reference position in the map space is larger than a threshold as a singular node.

### (Supplementary Note 11)

The control method according to Supplementary note 10,
wherein in the singular node detection step:
using a center of mass or a geometric center of the plurality of nodes that are assigned the material specification information as the reference position, and
using, as the threshold, a statistic value representing a magnitude of a distribution of positions of the plurality of nodes that are assigned the material specification information or a value obtained by multiplying the statistic value by a predetermined positive real number.

### (Supplementary Note 12)

The control method according to any one of Supplementary notes 8 to 11,
wherein in the singular node detection step:
dividing the plurality of the nodes that are assigned the material specification information into a plurality of clusters based on the material specification information; and
detecting, for each of the plurality of the clusters, the singular node out of the nodes belonging to the cluster based on a distribution of positions of the nodes in the map space included in the cluster.

### (Supplementary Note 13)

The control method according to any one of Supplementary notes 8 to 12, further comprising a map image generation step of generating a map image showing each of the nodes arranged in the map space,
wherein the map image includes an indication indicating the singular node.

### (Supplementary Note 14)

The control method according to Supplementary note 13,
wherein the indication indicating the singular node indicates one or both of the material specification represented by the material specification information assigned to the singular node and the physical properties represented by the physical property vector assigned to the singular node.

### (Supplementary Note 15)

A non-transitory computer readable medium for causing a computer to perform:
an acquisition step of acquiring, for each of a plurality of patterns of a material that can be used in a target process, material specification information representing a material specification of the material and physical property information indicating a physical property quantity for each of a plurality of physical properties of a product that can be generated in the target process by using the material;
a self-organizing map generation step of generating, by using the physical property information, a self-organizing map on which each node is assigned a position in a map space and a physical property vector indicating a value related to a physical property quantity for each of a plurality of types of the physical properties of the product; and
a singular node detection step of assigning each material specification information to one of the nodes based on the physical property information corresponding to that material specification information, and detecting a singular node located at a singular position in the map space out of the nodes that are assigned the material specification information.

### (Supplementary Note 16)

The non-transitory computer readable medium according to Supplementary note 15,
wherein in the singular node detection step, for each of a plurality of pieces of the material specification information, assigning the material specification information to the node to which the physical property vector that is most similar to the physical property vector obtained from the physical property information corresponding to that material specification information is assigned.

### (Supplementary Note 17)

The non-transitory computer readable medium according to Supplementary note 15 or 16,
wherein in the singular node detection step, detecting, out of the nodes that are assigned the material specification information, the node whose distance from a reference position in the map space is larger than a threshold as a singular node.

### (Supplementary Note 18)

The non-transitory computer readable medium according to Supplementary note 17,
wherein in the singular node detection step:
using a center of mass or a geometric center of the plurality of nodes that are assigned the material specification information as the reference position, and
using, as the threshold, a statistic value representing a magnitude of a distribution of positions of the plurality of nodes that are assigned the material specification information or a value obtained by multiplying the statistic value by a predetermined positive real number.

### (Supplementary Note 19)

The non-transitory computer readable medium according to any one of Supplementary notes 15 to 18,
wherein in the singular node detection step:
dividing the plurality of the nodes that are assigned the material specification information into a plurality of clusters based on the material specification information; and
detecting, for each of the plurality of the clusters, the singular node out of the nodes belonging to the cluster based on a distribution of positions of the nodes in the map space included in the cluster.

### (Supplementary Note 20)

The non-transitory computer readable medium according to any one of Supplementary notes 15 to 19, further comprising a map image generation step of generating a map image showing each of the nodes arranged in the map space,
wherein the map image includes an indication indicating the singular node.

### (Supplementary Note 21)

The non-transitory computer readable medium according to Supplementary note 20,
wherein the indication indicating the singular node indicates one or both of the material specification represented by the material specification information assigned to the singular node and the physical properties represented by the physical property vector assigned to the singular node.

This application is based upon and claims the benefit of priority from Japanese patent application No. 2021-044487, filed on March 18, 2021, the disclosure of which is incorporated herein in its entirety by reference.

### Reference Signs List

- 10: MATERIAL SPECIFICATION INFORMATION
- 20: PHYSICAL PROPERTY INFORMATION
- 30: SELF-ORGANIZING MAP
- 40: MAP IMAGE
- 42: INDICATION
- 44: REFERENCE POSITION
- 46: AREA
- 60: MATERIAL
- 70: PRODUCT
- 100: TABLE
- 102: MATERIAL IDENTIFICATION INFORMATION
- 104: MATERIAL SPECIFICATION
- 200: TABLE
- 202: PRODUCT IDENTIFICATION INFORMATION
- 204: PHYSICAL PROPERTY
- 300: TABLE
- 302: POSITION
- 304: PHYSICAL PROPERTY VECTOR
- 306: MATERIAL IDENTIFICATION INFORMATION
- 500: COMPUTER
- 502: BUS
- 504: PROCESSOR
- 506: MEMORY
- 508: STORAGE DEVICE
- 510: INPUT/OUTPUT INTERFACE
- 512: NETWORK INTERFACE
- 2000: SINGULAR MATERIAL DETECTION APPARATUS
- 2020: ACQUISITION UNIT
- 2040: SELF-ORGANIZING MAP GENERATION UNIT
- 2060: SINGULAR NODE DETECTION UNIT

## Claims

1. A singular material detection apparatus comprising:
acquisition means for acquiring, for each of a plurality of patterns of a material that can be used in a target process, material specification information representing a material specification of the material and physical property information indicating a physical property quantity for each of a plurality of physical properties of a product that can be generated in the target process by using the material;
self-organizing map generation means for generating, by using the physical property information, a self-organizing map on which each node is assigned a position in a map space and a physical property vector indicating a value related to a physical property quantity for each of a plurality of types of the physical properties of the product; and
singular node detection means for assigning each material specification information to one of the nodes based on the physical property information corresponding to that material specification information, and detecting a singular node located at a singular position in the map space out of the nodes that are assigned the material specification information.

2. The singular material detection apparatus according to claim 1,
wherein for each of a plurality of pieces of the material specification information, the singular node detection means assigns the material specification information to the node to which the physical property vector that is most similar to the physical property vector obtained from the physical property information corresponding to that material specification information is assigned.

3. The singular material detection apparatus according to claim 1 or 2,
wherein the singular node detection means detects, out of the nodes that are assigned the material specification information, the node whose distance from a reference position in the map space is larger than a threshold as a singular node.

4. The singular material detection apparatus according to claim 3,
wherein the singular node detection means performs:
using a center of mass or a geometric center of the plurality of nodes that are assigned the material specification information as the reference position; and
using, as the threshold, a statistic value representing a magnitude of a distribution of positions of the plurality of nodes that are assigned the material specification information are assigned or a value obtained by multiplying the statistic value by a predetermined positive real number.

5. The singular material detection apparatus according to any one of claims 1 to 4,
wherein the singular node detection means performs:
dividing the plurality of the nodes that are assigned the material specification information into a plurality of clusters based on the material specification information; and
detecting, for each of the plurality of the clusters, the singular node out of the nodes belonging to the cluster based on a distribution of positions of the nodes in the map space included in the cluster.

6. The singular material detection apparatus according to any one of claims 1 to 5, further comprising map image generation means for generating a map image showing each of the nodes arranged in the map space,
wherein the map image includes an indication indicating the singular node.

7. The singular material detection apparatus according to claim 6,
wherein the indication indicating the singular node indicates one or both of the material specification represented by the material specification information assigned to the singular node and the physical properties represented by the physical property vector assigned to the singular node.

8. A control method performed by a computer, comprising:
an acquisition step of acquiring, for each of a plurality of patterns of a material that can be used in a target process, material specification information representing a material specification of the material and physical property information indicating a physical property quantity for each of a plurality of physical properties of a product that can be generated in the target process by using the material;
a self-organizing map generation step of generating, by using the physical property information, a self-organizing map on which each node is assigned a position in a map space and a physical property vector indicating a value related to a physical property quantity for each of a plurality of types of the physical properties of the product; and
a singular node detection step of assigning each material specification information to one of the nodes based on the physical property information corresponding to that material specification information, and detecting a singular node located at a singular position in the map space out of the nodes that are assigned the material specification information.

9. The control method according to claim 8,
wherein in the singular node detection step, for each of a plurality of pieces of the material specification information, assigning the material specification information to the node to which the physical property vector that is most similar to the physical property vector obtained from the physical property information corresponding to that material specification information is assigned.

10. The control method according to claim 8 or 9,
wherein in the singular node detection step, detecting, out of the nodes that are assigned the material specification information, the node whose distance from a reference position in the map space is larger than a threshold as a singular node.

11. The control method according to claim 10,
wherein in the singular node detection step:
using a center of mass or a geometric center of the plurality of nodes that are assigned the material specification information as the reference position, and
using, as the threshold, a statistic value representing a magnitude of a distribution of positions of the plurality of nodes that are assigned the material specification information or a value obtained by multiplying the statistic value by a predetermined positive real number.

12. The control method according to any one of claims 8 to 11,
wherein in the singular node detection step:
dividing the plurality of the nodes that are assigned the material specification information into a plurality of clusters based on the material specification information; and
detecting, for each of the plurality of the clusters, the singular node out of the nodes belonging to the cluster based on a distribution of positions of the nodes in the map space included in the cluster.

13. The control method according to any one of claims 8 to 12, further comprising a map image generation step of generating a map image showing each of the nodes arranged in the map space,
wherein the map image includes an indication indicating the singular node.

14. The control method according to claim 13,
wherein the indication indicating the singular node indicates one or both of the material specification represented by the material specification information assigned to the singular node and the physical properties represented by the physical property vector assigned to the singular node.

15. A non-transitory computer readable medium for causing a computer to perform:
an acquisition step of acquiring, for each of a plurality of patterns of a material that can be used in a target process, material specification information representing a material specification of the material and physical property information indicating a physical property quantity for each of a plurality of physical properties of a product that can be generated in the target process by using the material;
a self-organizing map generation step of generating, by using the physical property information, a self-organizing map on which each node is assigned a position in a map space and a physical property vector indicating a value related to a physical property quantity for each of a plurality of types of the physical properties of the product; and
a singular node detection step of assigning each material specification information to one of the nodes based on the physical property information corresponding to that material specification information, and detecting a singular node located at a singular position in the map space out of the nodes that are assigned the material specification information.

16. The non-transitory computer readable medium according to claim 15,
wherein in the singular node detection step, for each of a plurality of pieces of the material specification information, assigning the material specification information to the node to which the physical property vector that is most similar to the physical property vector obtained from the physical property information corresponding to that material specification information is assigned.

17. The non-transitory computer readable medium according to claim 15 or 16,
wherein in the singular node detection step, detecting, out of the nodes that are assigned the material specification information, the node whose distance from a reference position in the map space is larger than a threshold as a singular node.

18. The non-transitory computer readable medium according to claim 17,
wherein in the singular node detection step:
using a center of mass or a geometric center of the plurality of nodes that are assigned the material specification information as the reference position, and
using, as the threshold, a statistic value representing a magnitude of a distribution of positions of the plurality of nodes that are assigned the material specification information or a value obtained by multiplying the statistic value by a predetermined positive real number.

19. The non-transitory computer readable medium according to any one of claims 15 to 18,
wherein in the singular node detection step:
dividing the plurality of the nodes that are assigned the material specification information into a plurality of clusters based on the material specification information; and
detecting, for each of the plurality of the clusters, the singular node out of the nodes belonging to the cluster based on a distribution of positions of the nodes in the map space included in the cluster.

20. The non-transitory computer readable medium according to any one of claims 15 to 19, further comprising a map image generation step of generating a map image showing each of the nodes arranged in the map space,
wherein the map image includes an indication indicating the singular node.

21. The non-transitory computer readable medium according to claim 20,
wherein the indication indicating the singular node indicates one or both of the material specification represented by the material specification information assigned to the singular node and the physical properties represented by the physical property vector assigned to the singular node.
